# EUROPÄISCHE PATENTSCHRIFT

(11) **EP 1 574 855 B1**
(45) Veröffentlichungstag und Bekanntmachung des Hinweises auf die Patenterteilung: **15.10.2014**
(21) Anmeldenummer: 04026397.2
(22) Anmeldetag: 01.12.2004
(51) Int. Cl.: G01N 33/487

(54) **Analysehandgerät**
Portable device for analysis
Appareil portable d'analyse

(30) Priorität: 23.12.2003 DE 10360786
(43) Veröffentlichungstag der Anmeldung: 14.09.2005
(73) Patentinhaber: Roche Diagnostics GmbH, 68305 Mannheim (DE); F. Hoffmann-La Roche AG, 4070 Basel (CH)
(72) Erfinder: Hübner, Ute, 64653 Lorsch (DE); Frey, Stephan Michael, 60433 Frankfurt (DE); Schabbach, Michael, 69469 Weiheim (DE)
(74) Vertreter: Jany und Petersen

(56) Entgegenhaltungen:
- EP-A- 0 732 590
- EP-A- 0 738 666
- US-A- 5 489 414
- US-A1- 2002 057 993

## Beschreibung

Die Erfindung betrifft ein tragbares Analysehandgerät zur Analyse eines medizinisch bedeutsamen Bestandteils einer Probe, insbesondere einer biologischen Flüssigkeit, mit einer Analysemeßeinrichtung, mit einem Gehäuse, das eine Ladeöffnung zum Aufnehmen eines auswechselbaren Trommelmagazins aufweist, das analytische Verbrauchsmittel, insbesondere Teststreifen, enthalten kann und mindestens eine Entnahmeöffnung an einer Stirnseite des Trommelmagazins aufweist, mit einem Deckel zum Verschließen der Ladeöffnung, mit einem Lager für das Trommelmagazin, das eine Rotation des Trommelmagazins um die geometrische Längsachse des Trommelmagazins ermöglicht, und mit einer Entnahmeeinrichtung zum Entnehmen eines analytischen Verbrauchsmittels aus dem Trommelmagazin, wobei das Gehäuse an einer Außenseite eine Ausgabeöffnung aufweist, durch die eines der Verbrauchsmittel mittels der Entnahmeeinrichtung geschoben werden kann.

Für die chemische und biochemische Analyse von festen und flüssigen Probenmaterialien haben sich in darauf spezialisierten Labors und insbesondere auch für den Einsatz außerhalb fester Labors trägergebundene Schnelltest etabliert. Solche trägergebundene Schnelltests basieren auf einer eigens entwickelten Trockenchemie und sind trotz der oftmals komplexen Reaktion unter Beteiligung empfindlicher Reagenzien selbst von Laien einfach und unkompliziert durchzuführen.

Ein bekanntes Beispiel für trägergebundene Schnelltests sind Testelemente für die Bestimmung des Blutglucosegehalts bei Diabetikern. Diagnostische Testelemente, die streifenförmig ausgebildet sind werden auch als Teststreifen bezeichnet. Bekannte Ausführungsformen sind z.B. sind Ein- oder Mehrfelder-Teststreifen für die Urinanalytik und diverse Indikatorpapiere. Da neben Testelementen in Streifenform auch andere Formen trägergebundener Tests existieren, spricht man allgemeiner von analytischen Verbrauchsmitteln.

Solche analytischen Verbrauchsmittel sind in einem Vorratsbehältnis verpackt, um sie vor schädlichen Umwelteinflüssen wie z.B. Licht, Feuchtigkeit oder mechanischer Einwirkung zu schützen und unter sterilen Bedingungen aufzubewahren. Zu analytischen Verbrauchsmitteln zählen neben Teststreifen beispielsweise auch Lanzetten oder Probenentnahmelemente.

Da derartige analytische Verbrauchsmittel im Stand der Technik umfassend beschrieben und dem Fachmann in einer Vielzahl von Ausführungsformen geläufig sind, erübrigt sich hier eine detaillierte Beschreibung. Die analytischen Verbrauchsmittel sind in einem Trommelmagazin gelagert, wie es beispielsweise in der EP 1 022 565 A2 beschrieben ist.

Tragbare Analysehandgeräte zur Analyse einer biologischen Flüssigkeit, wie beispielsweise Geräte zur Bestimmung des Blutglucosegehaltes, werden häufig von Personen verwendet, deren manuelle Geschicklichkeit wegen Krankheit oder Alter eingeschränkt ist. Es ist deshalb wichtig, daß sich solche Analysegeräte möglichst leicht handhaben lassen und Fehlbedienungen weitgehend ausgeschlossen sind. Als ein kritischer Punkt in diesem Zusammenhang hat sich das Einlegen oder Austauschen eines Trommelmagazins in das Analysegerät erwiesen. Einerseits muß dabei für eine genaue Positionierung der Trommel gesorgt werden, damit die im Trommelmagazin enthaltenen analytischen Verbrauchsmittel zuverlässig mit dem Analysesensor des Analysegeräts zusammenwirken können, andererseits darf der Austausch eines Trommelmagazins nur ein geringes Maß an manueller Geschicklichkeit vom Benutzer des Analysegeräts verlangen.

Im Handel erhältliche Analysehandgeräte haben einen Aufbau, wie er beispielsweise in der US 5,632,410 oder US 5,510,266 beschrieben ist. Bei einem solchen Analysegerät ist das Trommelmagazin auf einer Achse gelagert, die das Trommelmagazin auf voller Länge durchsetzt. Auf diese Weise läßt sich eine ausreichende Positionierung des Trommelmagazins erreichen. Nachteilig ist aber, daß der Austausch eines Trommelmagazins mühsam ist, da die Achse durch eine zentrale Bohrung des Trommelmagazins hindurchgeschoben werden muß, was ein nicht unerhebliches Maß an Fingerfertigkeit erfordert.

Ferner ist ein offenkundig vorbenutztes Analysegerät des Accu-Check®Compact Blood Glucose Systems bekannt (Reference Manual. Publ. Nr. 03307689001 (08/02), Roche Diagnostics, Mannheim, 2002). Das bekannte Gerät weist eine durch einen Deckel verschließbare Ladeöffnung zum Aufnehmen eines Trommelmagazins auf. In der Ladeöffnung befindet sich eine Achse, auf die das Trommelmagazin aufgesteckt wird. Der Deckel wird durch eine Schwenkbewegung geschlossen und drückt im geschlossenen Zustand auf eine Stirnseite des Trommelmagazins, wodurch es in seiner Arbeitsposition fixiert ist (vgl. Oberbegriff von Anspruch 1). Jedoch ist für einige Messungen eine präzisere axiale Positionierung wünschenswert. Außerdem kann das Trommelmagazin beim Öffnen des Deckels aus dem Gerät fallen, was lästig ist.

Aufgabe der Erfindung ist es daher, einen Weg aufzuzeigen, wie sich bei einem tragbaren Analysehandgerät die Handhabung, insbesondere ein Austausch eines dazugehörigen Trommelmagazins mit analytischen Verbrauchsmitteln, erleichtern läßt und zugleich eine korrekte Positionierung des Trommelmagazins in dem Analysehandgerät erreicht werden kann, so daß Verbrauchsmittel des Trommelmagazins mit der Analysemeßeinrichtung des Analysehandgeräts zusammenwirken können.

Diese Aufgabe wird von einem Analysegerät mit den Merkmalen des Anspruchs 1 gelöst.

Bei einem erfindungsgemäßen Analysehandgerät sind vorteilhaft das Positionieren des Trommelmagazins in seiner Arbeitsposition und das Schließen des Deckels in einem einzigen Handhabungsschritt zusammengefaßt. Ein Benutzer des erfindungsgemäßen Analysehandgeräts braucht sich um die korrekte Positionierung des Trommelmagazins nicht mehr zu kümmern, sondern verschließt nach dem Einlegen des Trommelmagazins, was ohne präzise Plazierung oder genaue Justage von Teilen erfolgen kann, einfach die Ladeöffnung des Analysehandgeräts mit dem Deckel, wodurch das Trommelmagazin positioniert wird. Dies ist gebrechlichen Menschen, deren manuelle Geschicklichkeit stark eingeschränkt ist, ohne Schwierigkeiten möglich.

Ein weiterer Vorteil eines erfindungsgemäßen Analysehandgerätes ist auch, daß durch das Schiebeteil eine genaue und zuverlässige axiale und radiale Positionierung des Trommelmagazins gewährleistet ist. Die Analysemeßeinrichtung kann deshalb zuverlässig mit in dem Trommelmagazin enthaltenen analytischen Verbrauchsmitteln zusammenwirken.

Insbesondere kann bei einem erfindungsgemäßen Analysehandgerät die axiale Positionierung des Trommelmagazins mit einer solchen Präzision erfolgen, daß - was bevorzugt ist - ein an dem Trommelmagazin angebrachter Code, z.B. ein Barcode zuverlässig mit einem Code-Leser gelesen werden kann. Auf diese Weise läßt sich beispielsweise ein Chargencode eines Trommelmagazins ablesen und eine darin enthaltene Information über die Verbrauchsmittel bei der Auswertung berücksichtigen. Ein fehlerhaft gelesener Code kann bei der Auswertung zu einem falschen Analyseergebnis führen. Deshalb muß der abstandsempfindliche Code-Leser sehr zuverlässig funktionieren, was eine präzise Positionierung des Trommelmagazins, wie sie bei einem erfindungsgemäßen Analysegerät gegeben ist, erforderlich macht. Insbesondere kann bei einem erfindungsgemäßen Analysegerät der Code-Leser mit seinen elektrischen Anschlüssen auch ortsfest innerhalb des Gehäuses des Analysegerätes angeordnet werden. Vorteilhaft kann dabei der Deckel von sämtlichen Komponenten des Code-Lesers freibleiben, was den Aufbau des Code-Lesers vereinfacht und seine Störanfälligkeit senkt.

Mittels des Schiebeteils kann bei einem erfindungsgemäßen Analysegerät auf eine das Trommelmagazin durchsetzende Achse verzichtet werden und trotzdem mittels einer Lagerung des Trommelmagazins an seinen gegenüberliegenden Enden in Verbindung mit dem Schiebeteil als Positioniereinrichtung eine präzise Positionierung erreicht werden. Insbesondere können über die Positioniereinrichtung, die bevorzugt das Trommelmagazin gegen das Lager drückt, auch Fertigungstoleranzen ausgeglichen werden, was eine kostengünstigere Fertigung erlaubt.

Ein erfindungsgemäßes Analysegerät ist tragbar und weist deshalb bevorzugt eine integrierte Stromquelle auf, so daß es netzunabhängig ist. Bevorzugt ist die Stromquelle als eine oder mehrere handelsübliche Batterien ausgebildet, kann aber beispielsweise auch Solarzellen umfassen.

Weitere Einzelheiten und Vorteile der Erfindung werden anhand von Ausführungsbeispielen unter Bezugnahme auf die beigefügten Figuren erläutert. Gleiche und einander entsprechende Teile sind dabei mit übereinstimmenden Bezugszahlen gekennzeichnet. Die darin dargestellten Besonderheiten können einzeln oder in Kombination verwendet werden, um bevorzugte Ausgestaltungen der Erfindung zu schaffen. Es zeigen:
- Fig. 1: ein Ausführungsbeispiel eines Analysehandgeräts,
- Fig. 2: den Deckel des in Fig. 1 gezeigten Ausführungsbeispiels,
- Fig. 3: das in Fig. 1 gezeigte Ausführungsbeispiel ohne oberen Gehäuseteil,
- Fig. 4: das in Fig. 1 gezeigte Ausführungsbeispiel ohne Gehäuse in einer Ansicht von unten,
- Fig. 5: die Positioniereinrichtung des in Fig. 1 bis 4 gezeigten Ausführungsbeispiels,
- Fig. 6: ein weiteres Ausführungsbeispiel der Positioniereinrichtung, und
- Fig. 7: ein weiteres Ausführungsbeispiel der Positioniereinerichtung.

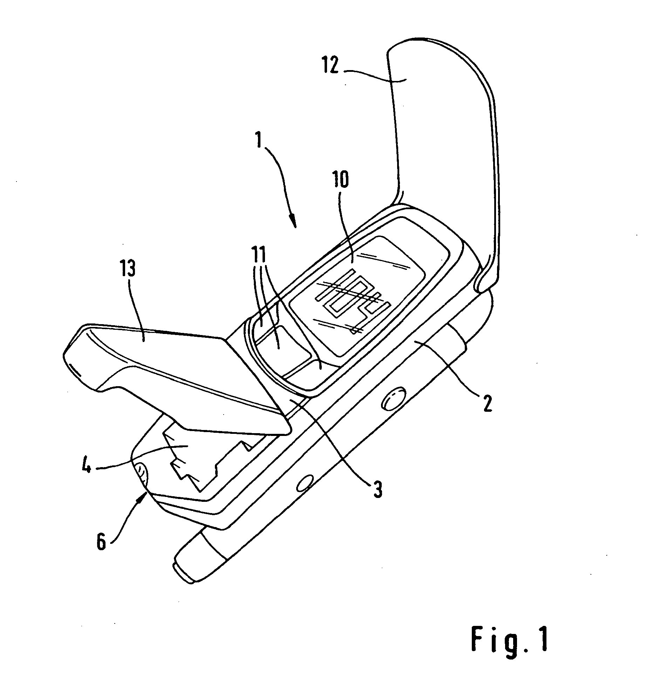

Fig. 1 zeigt ein Ausführungsbeispiel eines Analysegeräts, das als ein kompaktes, tragbares Analysehandgerät 1 ausgebildet ist und zur Analyse eines medizinisch bedeutsamen Bestandteils einer Probe dient, insbesondere einer biologischen Flüssigkeit, wie beispielsweise Blut, Blutplasma, Blutserum, Urin, Speichel, Sperma, Lymphflüssigkeit, Synovialflüssigkeit, Fruchtwasser, Tränenflüssigkeit, Zystenflüssigkeit, Schweißdrüsensekret oder Gallenflüssigkeit. Das Analysegerät 1 weist ein Gehäuse 2 auf, das in einer Längsseite 3 eine Ladeöffnung 4 zur Aufnahme eines auswechselbaren Trommelmagazins 5 hat.

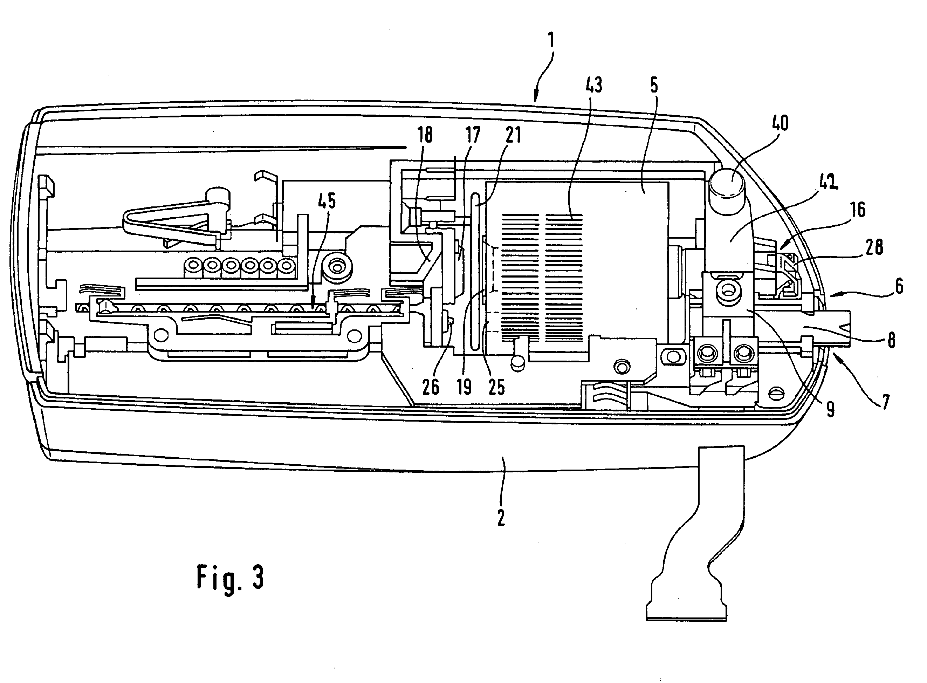

In einer Außenseite, bevorzugt in der Stirnseite 6 weist das Gehäuse 2 eine Ausgabeöffnung 7 für in dem Trommelmagazin 5 gelagerte analytische Verbrauchsmittel 8 (siehe Fig. 3) auf. Bevorzugt sind diese Verbrauchsmittel 8 als Teststreifen ausgebildet, auf die beispielsweise ein Tropfen Blut aufgebracht werden kann. Ein im Teststreifen enthaltenes Reagenz reagiert mit einem medizinisch wesentlichen Bestandteil der Probe, so daß das Ergebnis der Reaktion mit einer Analysemeßeinrichtung 9 des Analysegerätes 1 ausgewertet werden kann. Eine solche Analysemeßeinrichtung 9 kann beispielsweise ein optischer Sensor sein, der eine Farbänderung eines als Teststreifen ausgebildeten Verbrauchsmittels 8 erfaßt oder ein elektronischer Sensor, der eine Leitfähigkeitsänderung der Probe erfaßt.

Das Ergebnis einer Analyse wird mit einer Anzeigeeinrichtung 10, bevorzugt einem Flüssigkristalldisplay angezeigt. Der Anzeigeeinrichtung 10 benachbart sind Tasten 11 zur Bedienung des Analysegeräts 1 angeordnet. Zum Schutz dieser Tasten 11 und der Anzeigeeinrichtung 10 kann eine schwenkbare Abdeckung 12 an dem Gehäuse 2 angebracht sein.

Die Ladeöffnung 4 zur Aufnahme eines Trommelmagazins 5 ist mit einem Deckel 13 verschließbar, der schwenkbar am Gehäuse 2 befestigt ist.

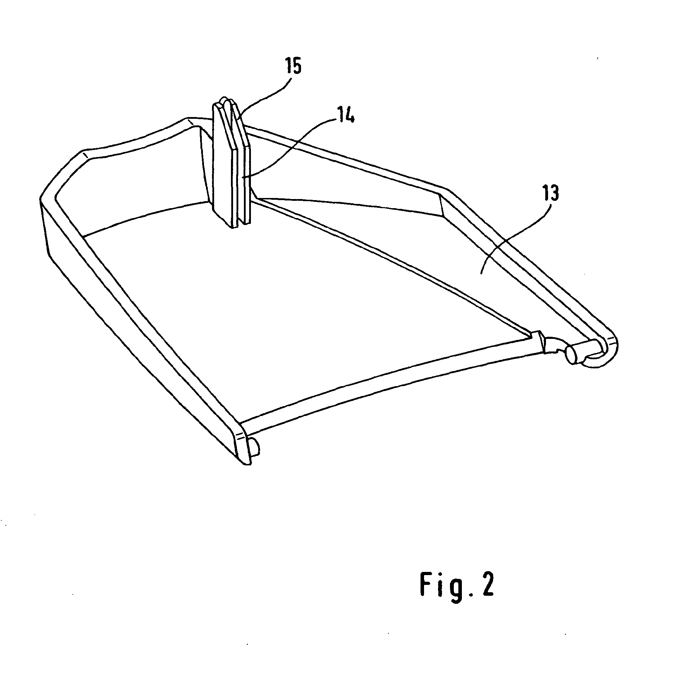

Der in Fig. 2 gezeigte Deckel 13 weist ein Positionierelement 14 auf, das im folgenden näher erläutert wird und so ausgebildet ist, daß das Trommelmagazin 5 beim Schließen des Deckels 13 in seine Arbeitsposition entlang seiner geometrischen Längsachse verschoben wird. Es wird also eine Schwenkbewegung des Deckels 13 durch das Positionierelement in eine Linearbewegung des Trommelmagazins umgesetzt.

Wie man in Fig. 1 sieht, ist der Deckel 13 im Bereich seines hinteren Endes am Gehäuse 2 befestigt und das Positionierelement 14 im Bereich des vorderen Endes des Deckels 13 angeordnet. Das Positionierelement 14 ist als ein Vorsprung ausgeführt, der bei geschlossenem Deckel 13 in das Innere des Gehäuses 2 hineinragt.

Wie man insbesondere in Fig. 2 sieht, weist das Positionierelement 14 eine Schrägfläche 15 auf. Beim Schließen des Deckels 13 liegt diese Schrägfläche 15 an der im folgenden näher erläuterten Positioniereinrichtung 16 an und drückt auf diese, wodurch die Schwenkbewegung des Deckels 13 in eine Längsbewegung, d.h. in Richtung der geometrischen Längsachse des Trommelmagazins 5, der Positioniereinrichtung 16 umgewandelt wird. Die Schrägfläche 15 ist bei geschlossenem Deckel 13 gegenüber der geometrischen Längsachse eines eingelegten Trommelmagazins 5 und damit auch gegenüber der Richtung, in welcher das Trommelmagazin 5 beim Schließen des Deckels 13 verschoben wird geneigt und einem eingelegten Trommelmagazin 5 zugewandt.

Fig. 3 zeigt das Analysegerät 1 bei abgenommenem oberen Gehäuseteil, so daß die Positioniereinrichtung 16 und das Trommelmagazin 5 in ihrer Arbeitsposition zu sehen sind. In der Arbeitsposition drückt die Positioniereinrichtung 16 das Trommelmagazin 5 gegen das Lager 17, das eine Rotation des Trommelmagazins 5 um seine geometrische Längsachse ermöglicht. Alternativ ist es aber auch möglich, daß die Positioniereinrichtung 16 das Trommelmagazin 5 ohne eine Anpreßkraft in der Arbeitsposition hält. Das Lager 17 weist einen Träger auf, der in eine axiale Ausnehmung 19 an einer Stirnseite des Trommelmagazins 5 eingreift und dessen Positionierung unterstützt. Mittels eines Elektromotors 20 kann das Trommelmagazin 5 über das an seiner Stirnseite anliegende Trommelrad 21 in Bewegung versetzt werden. Alternativ ist es auch möglich das Trommelmagazin 5 durch eine an seine Mantelfläche angreifende Walze in Rotation zu versetzen. Als Stromquelle 22 für den Elektromotor 20 dienen Batterien oder Akkumulatoren, wobei beispielsweise auch Solarzellen als Stromquelle 22 möglich sind.

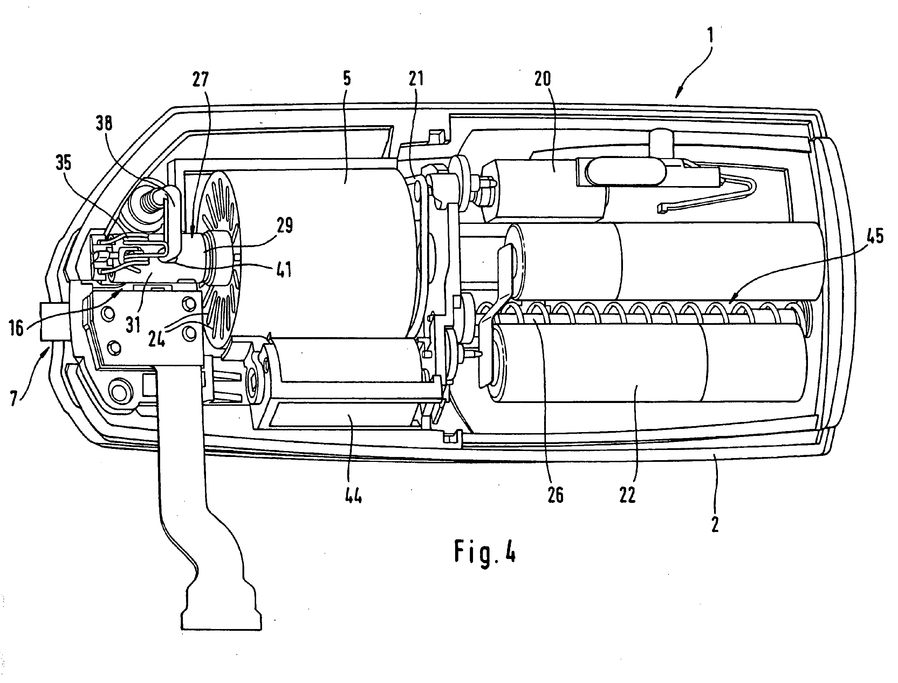

Das Trommelmagazin 5 hat mehrere ringförmig um seine geometrische Längsachse angeordnete Kammern 23, die analytische Verbrauchsmittel 8 enthalten können. Durch schrittweise Rotation des Trommelmagazins 5 können die Verbrauchsmittel 8 der Reihe nach bei Bedarf aus der jeweiligen Kammer 23 des Trommelmagazins 5 entnommen werden. Die Zahl dieser Kammern 23 kann weitgehend beliebig gewählt werden. In der Regel sind 10 bis 100 Kammern 23 zweckmäßig, bevorzugt sind 15 bis 30 Kammern 23 vorhanden. Wie man insbesondere in Fig. 4 erkennt, weist jede der Kammern 23 eine Entnahmeöffnung 24 zum Entnehmen eines Verbrauchsmittels 8 und eine der Entnahmeöffnung 24 gegenüberliegende Einschuböffnung 25 zum Einführen eines Stößels 26 einer Entnahmeeinrichtung 45, wie in der EP 1 022 565 A2 beschrieben, für den Transport des Verbrauchsmittels 8 auf. Die Einschub- und die Entnahmeöffnungen 24, 25 sind zum Schutz der Verbrauchsmittel 8 mit einer Schutzfolie verschlossen. Mit dem Stößel 26 lassen sich Verbrauchsmittel 8 zur Verwendung aus den Kammern 23 herausschieben, wobei die Schutzfolie durchstoßen wird. Je nach Größe und Form der Verbrauchsmittel 8 kann das Trommelmagazin 5 eine langgestreckte zylindrische Gestalt oder auch eine scheibenförmige Gestalt aufweisen.

Die Positioniereinrichtung 16 weist ein Schiebeteil 27 auf, welches das Trommelmagazin 5 beim Schließen des Deckels 13 in die in Fig. 3 gezeigte Arbeitsposition schiebt. Das Schiebeteil 27 weist eine Schrägfläche 28 auf, an der das Positionierelement 14 beim Schließen des Deckels 13 angreift. Dabei drückt die Schrägfläche 15 des Positionierelements 14 gegen die Schrägfläche 28 des Schiebeteils 27, wodurch eine Schwenkbewegung des Deckels 13 in eine Linearbewegung des Schiebeteils 27 umgesetzt wird. Das Schiebeteil 27 schiebt dann das Trommelmagazin 5 entlang seiner Längsachse in die Arbeitsposition und drückt es gegen das Lager 17. Dieses Verschieben des Trommelmagazins 5 kann bei einer entsprechenden Führung des Schiebeteils mit einem kleinen Rotationsschritt des Trommelmagazins verbunden sein.

Das Schiebeteil 27 weist einen Zapfen 29 auf, der in eine axiale Ausnehmung 30 des Trommelmagazins 5 eingreift und so für eine axiale Positionierung des Trommelmagazins 5 sorgt.

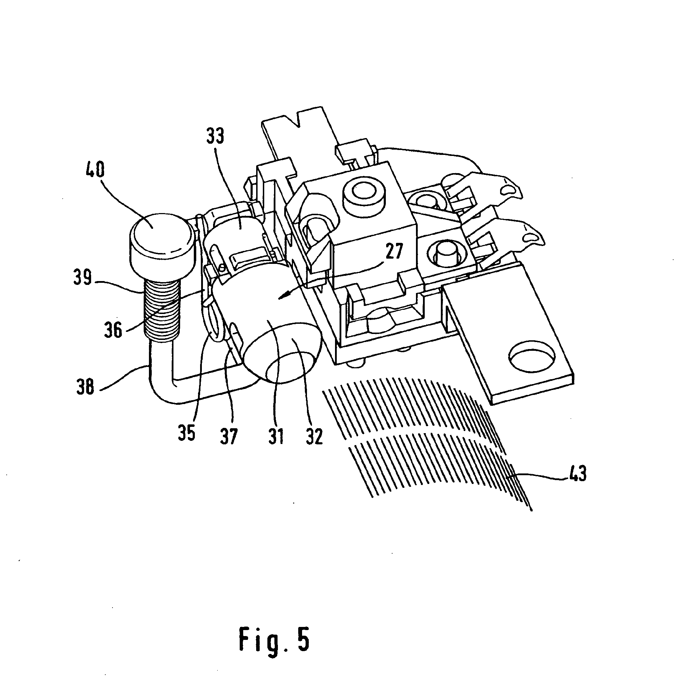

Fig. 4 zeigt die Positioniereinrichtung 16 und das Trommelmagazin 5 bei abgenommenem Gehäuse 2 des Analysegeräts 1 in einer Ansicht von unten. Das Schiebeteil 27 weist eine äußere Hülse 31 auf, die mit ihrem vorderen Ende den Zapfen 29 ausbildet. Um einen positionsgenauen Eingriff des Zapfens 29 in die axiale Ausnehmung 30 des Trommelmagazins 5 zu verbessern ist der Zapfen 29 bevorzugt mit einer Fase 32 versehen oder mit seinem vorderen, eingreifenden Ende konusförmig ausgeführt, was insbesondere in Fig. 5 zu sehen ist.

In Fig. 5 ist die Positioniereinrichtung 16 im Detail gezeigt, wobei ein Teil der äußeren Hülse 31 entfernt ist, so daß der Aufbau des Schiebeteils 27 besser erkennbar ist. In der Hülse 31 des Schiebeteils 27 befindet sich ein Kolben 32 mit einer Schraubenfeder 34, die mit einem Ende gegen die äußere Hülse 31 und mit ihrem anderen Ende gegen den Kolben 32 drückt. Der Kolben 32 ist gegenüber der äußeren Hülse 31 des Schiebeteils 27 beweglich, so daß die Länge des Schiebeteils 27 veränderlich ist. Die Feder 34 ist also zwischen dem Zapfen 29 und der Schrägfläche 28 angeordnet. Mittels der Feder 34 können so Fertigungstoleranzen in axialer Richtung ausgeglichen werden. Der Kolben 32 weist an seinem von der äußeren Hülse 31 abgewandten Ende die erwähnte Schrägfläche 28 der Positioniereinrichtung 16 auf, die mit dem am Deckel 13 angebrachten Positionierelement 14 zusammenwirkt.

Wie man insbesondere in Fig. 5 erkennt, weist die Positioniereinrichtung 16 eine zweite Feder 35 auf, die als eine Schenkelfeder ausgebildet ist. Die zweite Feder 35 ist relativ zum Gehäuse 2 ortsfest abgestützt und drückt gegen das Positionierelement 14, so daß das Schiebeteil 27 beim Öffnen des Deckels zurückgesetzt wird und das Trommelmagazin 5 entnommen werden kann. Die zweite Feder 35 weist einen ersten Schenkel 36 auf, mit dem sie gegen das Schiebeteil 27 drückt. Die Feder 35 stützt sich mit einem zweiten Schenkel 37 relativ zum Gehäuse 2 ortsfest ab.

Um das am Deckel 13 angeordnete Positionierelement 14 von dem in Längsrichtung des Trommelmagazins 5 wirkenden Druck der beiden Federn 34, 35 zu entlasten, ist ein Fixierelement 38 vorhanden, mittels dem das Schiebeteil 27 in seiner in Fig. 3 gezeigten Position fixierbar ist. Dieses Fixierelement 38 verhindert dabei insbesondere auch, daß bei einem Öffnen des Deckels 13 das Schiebeteil 27 oder das Trommelmagazin 5 unkontrolliert aus der Ladeöffnung 4 des Analysegeräts 1 springen. Mittels des Fixierelement 38 ist das Schiebeteil 27 fixierbar, wenn es das Trommelmagazin 5 in seine in Fig. 3 gezeigte Arbeitsposition gebracht hat.

Bevorzugt tritt das Fixierelement 38 formschlüssig mit dem Schiebeteil 27 in Eingriff, da ein Formschluß gegenüber einem ebenfalls möglichen Kraftschluß eine zuverlässigere Sicherung darstellt. Wie man insbesondere in Fig. 5 erkennt, ist das Fixierelement 38 mittels der Feder 39 federgelagert und U-förmig um das Schiebeteil herum angeordnet. Durch manuelles Drücken auf ein Ende des Fixierelementes 38, das bei dem gezeigten Ausführungsbeispiel zur besseren Handhabung mit einem Knopf 40 versehen ist, wird das andere Ende des Fixierelementes 38 von dem Schiebeteil 27 abgehoben und so aus seinem Eingriff mit einer Raststelle 41 des Schiebeteils 27 gelöst.

Die als Schraubenfeder ausgebildete Feder 39, die um das Fixierelement 38 herum angeordnet ist, stützt sich an dem Trägersteg 42 ab und drückt so das Fixierelement 38 gegen das Schiebeteil 27.

Die Raststelle 41 des Schiebeteils 27 ist als ein Sackloch ausgebildet. Wird das Schiebeteil 27 beim Schließen des Deckels 13 mit der Raststelle 41 an dem Fixierelement 38 vorbeigeschoben, so wird das Fixierelement 38 von der Feder 39 in die Raststelle 41 gedrückt, so daß ein Formschluß entsteht.

Wie man insbesondere in Fig. 3 erkennt, kann das Trommelmagazin 5 einen Barcode 43 aufweisen. Sind die Verbrauchsmittel 8 beispielsweise als Teststreifen zur Bestimmung des Blutglucosegehaltes ausgebildet, so kann ein im Barcode 43 enthaltener Chargencode bei der Auswertung eines vom Teststreifen erzeugten Farbsignales berücksichtigt werden. Ein gegenüber dem Gehäuse ortsfest angeordneter Barcode-Leser 44 kann beim Einlegen eines neuen Trommelmagazins 5 automatisch den Barcode 43 lesen, so daß darin enthaltene Informationen bei der Auswertung eines Meßergebnisses berücksichtigt werden können. Vorteilhaft kann so die Meßgenauigkeit verbessert werden ohne, daß eine entsprechende Information von Hand eingetippt werden muß. Die präzise Positionierung eines Trommelmagazins 5 in dem beschriebenen Analysegerät 1 erlaubt vorteilhaft den Einsatz abstandsempfindlicher Barcode-Leser 44, ohne daß Lesefehler zu befürchten sind.

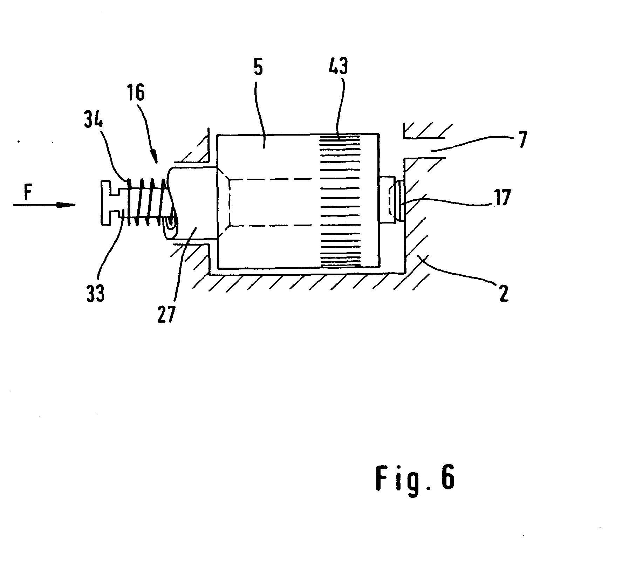

Bei dem anhand der Fig. 1 bis 5 beschriebenen Ausführungsbeispiel wird das Trommelmagazin 5 beim Schließen des Deckels 13 von der Ausgabeöffnung 7 des Analysegeräts 1 weg nach hinten auf den feststehenden Träger 18 des Lagers 17 verschoben. Die Schenkelfeder 35 wird beim Schließen des Deckels 13 mit Druck belastet und entspannt sich beim Öffnen. Dieser Aufbau ist aber nicht zwingend. Ebenso gut ist es möglich, daß das Trommelmagazin 5 beim Schließen des Deckels 13 zur Stirnseite 6 des Analysegeräts 1 mit der Ausgabeöffnung 7 hin verschoben wird, wie in Fig. 6 gezeigt. Bei einem solchen alternativen Aufbau sind das Schiebeteil 27 und der Träger 18 des Lagers 17 mit dem Trommelrad 21 zum Antreiben des Trommelmagazins 5 auf der jeweils anderen Seite angeordnet, als es bei dem anhand der Figuren 1 bis 5 beschriebenen Ausführungsbeispiel der Fall ist.

Bei dem in Fig. 6 gezeigten Ausführungsbeispiel ist die Feder 35 so angeordnet, daß diese beim Öffnen des Deckels 13 mit Zug beansprucht wird, beispielsweise indem das Positionierelement 14 in das Schiebeteil 27 eingreift und dieses zieht. Beim Schließen des Deckels 13 zieht die Feder 35 dann das Schiebeteil 27 zurück, so daß das Trommelmagazin 5 in seiner Arbeitsposition gehalten ist.

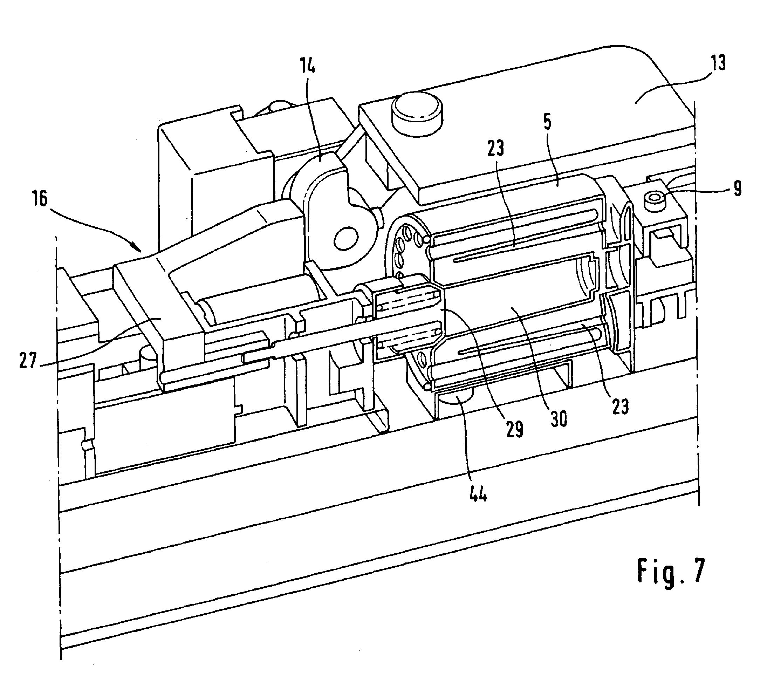

Fig. 7 zeigt im Detail, wie der anhand von Fig. 6 beschriebene alternative Aufbau, bei dem das Trommelmagazin 5 beim Schließen des Deckels 13 zur Ausgabeöffnung 7 hin verschoben wird, verwirklicht werden kann. Bei dem in Fig. 7 mit eingelegtem Trommelmagazin 5 und geschlossenem Deckel 13 gezeigten Ausführungsbeispiel ist das Positionierelement 14 als eine Kurvenscheibe ausgebildet, die an einem Ausleger des Deckels 13 angebracht ist, über den der Deckel 13 gelenkig mit dem Gehäuse 2 des Analysegeräts 1 verbunden ist. Das Schiebeteil 27 ist U-förmig ausgebildet und wird durch Federkraft gegen das als Kurvenscheibe ausgebildete Positionierelement 14 gedrückt. Dabei liegt ein Schenkel des U-förmigen Schiebeteils 27 an der Kurvenscheibe 14 an, während der andere Schenkel gegen das Trommelmagazin 5 drückt.

Wird der Deckel 13 geöffnet, so dreht sich die Kurvenscheibe 14. Dabei wird durch die von der Kreisform abweichende Außenfläche der Kurvenscheibe 14 das Schiebeteil 27 gegen Federkraft nach hinten gedrückt, d.h. weg von der Ausgabeöffnung 7. Dabei löst sich das Schiebeteil 27 aus seinem Eingriff in die axiale Ausnehmung 30 des Trommelmagazins 5, so daß dieses aus dem geöffneten Analysegerät 1 entnommen und ein neues Trommelmagazin 5 eingelegt werden kann. Wird anschließend der Deckel 13 wieder geschlossen, so nimmt die Kurvenscheibe 14 wieder die in Fig. 7 gezeigte Position ein und das Schiebeteil 27 kann durch Federkraft nach vorn bewegt werden. Dabei tritt das U-förmig ausgebildete Schiebeteil 27 mit einem seiner Schenkel mit dem Trommelmagazin 5 in Eingriff und schiebt dieses in seine Arbeitsposition.

### Bezugszeichenliste

- 1: Analysehandgerät
- 2: Gehäuse
- 3: Längsseite
- 4: Ladeöffnung
- 5: Trommelmagazin
- 6: Stirnseite des Analysehandgeräts
- 7: Ausgabeöffnung des Gehäuses
- 8: Verbrauchsmittel
- 9: Analysemeßeinrichtung
- 10: Anzeigeeinrichtung
- 11: Tasten
- 12: Abdeckung
- 13: Deckel
- 14: Positionierelement
- 15: Schrägfläche des Positionierelements
- 16: Positioniereinrichtung
- 17: Lager
- 18: Träger
- 19: Ausnehmung des Trommelmagazins
- 20: Elektromotor
- 21: Trommelrad
- 22: Stromquelle
- 23: Kammern
- 24: Entnahmeöffnung des Trommelmagazins
- 25: Einschuböffnung
- 26: Stößel
- 27: Schiebeteil
- 28: Schrägfläche des Schiebeteils
- 29: Zapfen
- 30: Ausnehmung des Trommelmagazins
- 31: Hülse
- 32: Fase
- 33: Kolben
- 34: Feder
- 35: Feder
- 36: erster Schenkel
- 37: zweiter Schenkel
- 38: Fixierelement
- 39: Feder
- 40: Knopf
- 41: Raststelle
- 42: Trägersteg
- 43: Barcode
- 44: Barcode-Leser
- 45: Entnahmeeinrichtung

## Patentansprüche

1. Tragbares Analysehandgerät zur Analyse eines medizinisch bedeutsamen Bestandteils einer Probe, insbesondere einer biologischen Flüssigkeit,
mit einem Analysemeßeinrichtung (9),
mit einem Gehäuse (2), das eine Ladeöffnung (4) zum Aufnehmen eines auswechselbaren Trommelmagazins (5) aufweist, das analytische Verbrauchsmittel (8), insbesondere Teststreifen, enthalten kann und mindestens eine Entnahmeöffnung (24) an einer Stirnseite des Trommelmagazins (5) aufweist,
mit einem Deckel (13) zum Verschließen der Ladeöffnung (4),
mit einem Lager (17) für das Trommelmagazin (5), das eine Rotation des Trommelmagazins (5) um die geometrische Längsachse des Trommelmagazin (5) ermöglicht, und
mit einer Entnahmeeinrichtung (45) zum Entnehmen eines der analytischen Verbrauchsmittel (8) aus dem Trommelmagazin (5), wobei das Gehäuse (2) an einer Außenseite eine Ausgabeöffnung (7) aufweist, durch die eines der Verbrauchsmittel (8) mittels der Entnahmeeinrichtung (45) geschoben werden kann,
**dadurch gekennzeichnet, daß**
der Deckel (13) an seiner der Ladeöffnung (4) zugewandten Seite ein Positionierelement (14) aufweist, das derart ausgebildet ist, dass es beim Schließen des Deckels (13) auf ein Schiebeteil (27) einwirkt und dadurch eine Linearbewegung des Schiebeteils (27) bewirkt, die das Trommelmagazin (5) in eine Arbeitsposition bewegt, in der es um seine geometrische Längsachse rotierbar ist.

2. Analysehandgerät nach Anspruch 1, **dadurch gekennzeichnet, daß** das Schiebeteil (27) in eine axiale Ausnehmung (30) des Trommelmagazins (5) eingreift.

3. Analysehandgerät nach einem der vorhergehenden Ansprüche, **dadurch gekennzeichnet, daß** es ein Fixierelement (38) umfaßt, mittels dem das Schiebeteil (27) fixierbar ist.

4. Analysehandgerät nach Anspruch 3, **dadurch gekennzeichnet, daß** das Schiebeteil (27) so fixierbar ist, daß es das Trommelmagazin (5) in der Arbeitsposition rotierbar arretiert.

5. Analysehandgerät nach Anspruch 3 oder 4, **dadurch gekennzeichnet, daß** das Schiebeteil (27) eine Raststelle (41) aufweist, die formschlüssig mit dem Fixierelement in Eingriff treten kann.

6. Analysehandgerät nach Anspruch 3, 4 oder 5, **dadurch gekennzeichnet, daß** das Fixierelement (38) federgelagert ist.

7. Analysehandgerät nach einem der Ansprüche 3 bis 6, **dadurch gekennzeichnet, daß** das Fixierelement (38) U-förmig um das Schiebeteil (27) herum angeordnet ist, so daß durch Druck auf ein Ende des Fixierelementes (38) sein anderes Ende aus einem Eingriff mit dem Schiebeteil (27) lösbar ist.

8. Analysehandgerät nach einem der vorhergehenden Ansprüche, **dadurch gekennzeichnet, daß** das Schiebeteil (27) in Richtung der geometrischen Längsachse eines eingelegten Trommelmagazins (5) verschieblich ist.

9. Analysehandgerät nach einem der vorhergehenden Ansprüche, **dadurch gekennzeichnet, daß** der Deckel (13) schwenkbar am Gehäuse (2) befestigt ist.

10. Analysehandgerät nach einem der vorhergehenden Ansprüche, **dadurch gekennzeichnet, daß** das Schiebeteil (27) Teil einer Positioniereinrichtung (16) ist, zu der eine Feder (34) gehört, die auf das Schiebeteil (27) in Längsrichtung eine Kraft ausübt und Fertigungstoleranzen in axialer Richtung ausgleicht.

11. Analysehandgerät nach Anspruch 10, **dadurch gekennzeichnet, daß** die Feder (34) eine Schraubenfeder ist.

12. Analysehandgerät nach einem der vorhergehenden Ansprüche, **dadurch gekennzeichnet, daß** das Lager (17) einen Träger (18) aufweist, der in eine axiale Ausnehmung (19) des Trommelmagazins (5) eingreift.

13. Analysehandgerät nach einem der vorhergehenden Ansprüche, **dadurch gekennzeichnet, daß** es einen ortsfest innerhalb des Gehäuses (2) angeordneten Lesesensor (44) zum Ablesen einer Kodierung (43) auf der Mantelfläche des Trommelmagazins (5) aufweist.

14. Analysehandgerät nach einem der vorhergehenden Ansprüche, **dadurch gekennzeichnet, daß** die Ladeöffnung (4) in einer Längsseite (3) des Gehäuses (2) angeordnet ist.

## Claims

1. Portable hand-held analytical device for analysis of a medically significant component of a sample, in particular a biological fluid,
with an analytical measuring unit (9),
with a housing (2) comprising a loading opening (4) for receiving a replaceable drum cartridge (5), which can contain analytical consumables (8), in particular test strips, and comprises at least one removal opening (24) at a front face of the drum cartridge (5),
with a lid (13) for closing the loading opening (4),
with a bearing (17) for the drum cartridge (5) facilitating a rotation of the drum cartridge (5) about the geometric longitudinal axis of the drum cartridge (5), and
with a removal facility (45) for removing an analytical consumable (8) from the drum cartridge (5), whereby the housing (2) comprises on an outer side a dispensing opening (7) through which a consumable (8) can be pushed by means of the removal facility (45),
**characterized in that**
the lid (13) comprises a positioning element (14) on the site facing the loading opening (4), which positioning element (14) is designed such that, upon closing of the lid (13), it effects a linear motion of a pushing part (27) which moves the drum cartridge (5) to a working position, in which it can be rotated about its geometric longitudinal axis.

2. Hand-held analytical device according to claim 1, **characterized in that** the pushing part (27) engages an axial recess (30) of the drum cartridge (5).

3. Hand-held analytical device according to any one of the preceding claims, **characterized in that** it comprises a fixing element (38) by means of which the pushing part (27) can be fixed in place.

4. Hand-held analytical device according to claim 3, **characterized in that** the pushing part (27) can be fixed in place such that it locks the drum cartridge (5) in the working position capable of rotating.

5. Hand-held analytical device according to claim 3 or 4, **characterized in that** the pushing part (27) comprises a lock-in position (41), which can engage the fixing element in a positive locking fashion.

6. Hand-held analytical device according to claim 3, 4 or 5, **characterized in that** the fixing element (38) is borne on springs.

7. Hand-held analytical device according to any one of the claims 3 to 6, **characterized in that** the fixing element (38) is arranged in a U-shape around the pushing part (27) such that by applying pressure to one end of the fixing element (38) its other end can be loosened from engagement with the pushing part (27).

8. Hand-held analytical device according to any one of the preceding claims, **characterized in that** the pushing part (27) can be pushed in the direction of the geometric longitudinal axis of an inserted drum cartridge (5).

9. Hand-held analytical device according to any one of the preceding claims, **characterized in that** the lid (13) is attached to the housing (2) so as to be capable of swiveling.

10. Hand-held analytical device according to any one of the preceding claims, **characterized in that** the pushing part (27) is part of a positioning facility (16) which includes a spring (34), which exerts a pressure in longitudinal direction on the pushing part (27) and compensates for manufacturing tolerances in axial direction.

11. Hand-held analytical device according to claim 10, **characterized in that** the spring (34) is a helical spring.

12. Hand-held analytical device according to any one of the preceding claims, **characterized in that** the bearing (17) comprises a carrier (18) which engages an axial recess (19) of the drum cartridge (5).

13. Hand-held analytical device according to any one of the preceding claims, **characterized in that** it comprises a reader sensor (44) for reading a code (43) on the jacket surface of the drum cartridge (5), which reader sensor (44) is arranged fixed in place inside the housing (2).

14. Hand-held analytical device according to any one of the preceding claims, **characterized in that** the loading opening (4) is arranged in a longitudinal side (3) of the housing (2).

## Revendications

1. Appareil portable pour analyses servant à l'analyse d'un composant - médicalement important - d'un échantillon, en particulier d'un liquide biologique, ledit appareil portable pour analyses comprenant un dispositif de mesure d'analyse (9),
un boîtier (2) qui présente une ouverture de chargement (4) servant à recevoir un magasin à tambour (5) interchangeable qui peut contenir des consommables (8) analytiques, en particulier des bandes de tests, et présente au moins une ouverture de prélèvement (24) située sur un côté frontal du magasin à tambour (5),
un couvercle (13) servant à fermer l'ouverture de chargement (4),
un palier (17) pour le magasin à tambour (5), lequel palier permet une rotation du magasin à tambour (5) autour de l'axe longitudinal géométrique du magasin à tambour (5), et
un dispositif de prélèvement (45) servant à prélever l'un des consommables analytiques (8) du magasin à tambour (5), où le boîtier (2) présente, sur un côté extérieur, une ouverture de sortie (7) à travers laquelle l'un des consommables (8) peut être poussé au moyen du dispositif de prélèvement (45),
**caractérisé en ce que**
le couvercle (13) présente, sur son côté tourné vers l'ouverture de chargement (4), un élément de positionnement (14) qui est conçu de manière telle, qu'il agisse sur une pièce coulissante (27) lors de la fermeture du couvercle (13) et, de ce fait, provoque un mouvement linéaire de la pièce coulissante (27), mouvement linéaire qui déplace le magasin à tambour (5) dans une position de travail dans laquelle ledit magasin à tambour peut tourner autour de son axe longitudinal géométrique.

2. Appareil portable pour analyses selon la revendication 1, **caractérisé en ce que** la pièce coulissante (27) pénètre dans un évidement axial (30) du magasin à tambour (5).

3. Appareil portable pour analyses selon l'une des revendications précédentes, **caractérisé en ce qu'**il comprend un élément de fixation (38) au moyen duquel peut être fixée la pièce coulissante (27).

4. Appareil portable pour analyses selon la revendication 3, **caractérisé en ce que** la pièce coulissante (27) est fixée de manière telle qu'elle arrête le magasin à tambour (5) de tourner dans la position de travail.

5. Appareil portable pour analyses selon la revendication 3 ou 4, **caractérisé en ce que** la pièce coulissante (27) présente un point d'encliquetage (41) qui peut venir en prise avec l'élément de fixation, par complémentarité de forme.

6. Appareil portable pour analyses selon la revendication 3, 4 ou 5, **caractérisé en ce que** l'élément de fixation (38) est à ressort.

7. Appareil portable pour analyses selon l'une quelconque des revendications 3 à 6, **caractérisé en ce que** l'élément de fixation (38) est disposé en forme de U tout autour de la pièce coulissante (27), de sorte que, par pression exercée sur une extrémité de l'élément de fixation (38), son autre extrémité peut être détachée d'une prise avec la pièce coulissante (27).

8. Appareil portable pour analyses selon l'une quelconque des revendications précédentes, **caractérisé en ce que** la pièce coulissante (27) est mobile en direction de l'axe longitudinal géométrique d'un magasin à tambour (5) encastré.

9. Appareil portable pour analyses selon l'une quelconque des revendications précédentes, **caractérisé en ce que** le couvercle (13) est fixé en pouvant pivoter sur le boîtier (2).

10. Appareil portable pour analyses selon l'une quelconque des revendications précédentes, **caractérisé en ce que** la pièce coulissante (27) fait partie d'un dispositif de positionnement (16) dont fait partie un ressort (34) qui exerce une force sur la pièce coulissante (27), dans la direction longitudinale, et compense des tolérances de fabrication dans la direction axiale.

11. Appareil portable pour analyses selon la revendication 10, **caractérisé en ce que** le ressort (34) est un ressort hélicoïdal.

12. Appareil portable pour analyses selon l'une quelconque des revendications précédentes, **caractérisé en ce que** le palier (17) présente un support (18) qui pénètre dans un évidement axial (19) du magasin à tambour (5).

13. Appareil portable pour analyses selon l'une quelconque des revendications précédentes, **caractérisé en ce qu'**il présente un capteur de lecture (44) disposé fixement à l'intérieur du boîtier (2) et servant à lire un codage (43) sur la surface latérale du magasin à tambour (5).

14. Appareil portable pour analyses selon l'une quelconque des revendications précédentes, **caractérisé en ce que** l'ouverture de chargement (4) est disposée dans un côté longitudinal (3) du boîtier (2).
